Europäisches Patentamt

(19) European Patent Office

Office européen des brevets

(11) Veröffentlichungsnummer: **0 019 115**
**B1**

(12) **EUROPÄISCHE PATENTSCHRIFT**

(45) Veröffentlichungstag der Patentschrift:
12.01.83

(51) Int. Cl.³: **C 07 C 103/52**, C 07 C 141/16,
A 61 K 37/02, A 61 K 37/24

(21) Anmeldenummer: 80102213.8

(22) Anmeldetag: 24.04.80

(54) Pankreozymin-Cholezystokinin aktive Peptide, Verfahren zu ihrer Herstellung und sie enthaltende Arzneimittel.

(30) Priorität: 30.04.79 DE 2917603
25.10.79 DE 2943132

(43) Veröffentlichungstag der Anmeldung:
26.11.80 Patentblatt 80/24

(45) Bekanntmachung des Hinweises auf die Patenterteilung:
12.01.83 Patentblatt 83/2

(84) Benannte Vertragsstaaten:
AT CH DE FR GB LI SE

(56) Entgegenhaltungen:
DE-A-1 800 129
DE-A-1 922 185
DE-A-1 935 402
DE-A-2 037 697
DE-A-2 256 445
DE-A-2 727 048

(73) Patentinhaber: Max-Planck-Gesellschaft zur Förderung der Wissenschaften e.V., Bunsenstrasse 10,
D-3400 Göttingen (DE)

(72) Erfinder: Wünsch, Erich, Dr. Prof., Midgardstrasse 16,
D-8132 Tutzing (DE)

(74) Vertreter: Lederer, Franz, Dr. et al, Patentanwälte Dr.
Franz Lederer Dipl.-Ing. Reiner F. Meyer-Roxlau
Lucile-Grahn-Strasse 22, D-8000 München 80 (DE)

Pankreozymincholezystokinin aktive Peptide, Verfahren zu ihrer Herstellung und sie enthaltende Arzneimittel

Die Erfindung betrifft Nonapeptide mit der carboxylendständigen Sequenz des Pankreozymincholezystokinins 25-33 dergestalt, dass der Aminosäurerest 25 eine stark basische Gruppe trägt, der Aminosäurerest 28 einen hydrophilen Charakter vom Typ Hydroxyaminosäuren, wie z.B. Threonin, Serin, Hydroxy-Prolin, aufweist und der Aminosäurerest 31 einen stark hydrophoben Charakter vom Typ Aminosäure mit aliphatischen Seitenketten, wie z.B. Norleucin, Norvalin und $\alpha$-Aminobuttersäure, besitzt. Entscheidend für die beiden letzten Variationen der natürlichen Sequenz ist, dass der „Gesamt-Polaritäts-Charakter", bedingt durch die im Naturstoff vorhandenen Methioninreste, durch obengenannten spezifischen Austausch in der Grösse erhalten bleibt.

Die erfindungsgemässen Nonapeptide besitzen die allgemeine Formel H-X-Asp-Tyr($SO_3H$)-Y-Gly-Trp-Z-Asp-Phe-$NH_2$, wobei X Arginin, Homoarginin, Norarginin, $N_\varepsilon$, $N_\varepsilon$-Dialkyllysin, $N_\delta$ oder $N_\delta$-Dialkylornithin, Y Threonin, Serin oder Hydroxyprolin und Z Norleucin, Norvalin oder $\alpha$-Aminobuttersäure, oder, weniger bevorzugt, Leucin ist. Eine bevorzugte Sequenz ist H-Arg-Asp-Tyr($SO_3H$)-Thr-Gly-Trp-Nle-Asp-Phe-$NH_2$.

Die Erfindung betrifft auch die Verwendung der erfindungsgemässen peptide als Pankreozymincholezystokininaktive Verbindungen.

Die erfindungsgemässen Peptide besitzen eine höhere Altivität als das Pankreozymin. Sie eignen sich zur Verwendung in Arzneimitteln zur Steuerung der Funktion der Gallenblase und zur Steuerung der Enzymsekretion des Pankreas.

Der Aminosäurerest 28 hat zur vollen Erhaltung der Hormonaktivität ein gegenüber dem natürlichen Bausteinmethionin hydrophilerer Rest zu sein, wie z.B. Threonin, Serin oder Hydroxyprolin, während der Aminosäurerest 31, gegenüber dem natürlichen Bausteinmethionin, ein hydrophoberer Aminosäurerest sein muss, wie z.B. Norleucin, Norvalin oder $\alpha$-Aminobuttersäure.

Der gleichzeitig vorzunehmende Austausch der Positionen 28 und 31 des Naturstoffes, d.i. die Besetzung der Nonapeptidsequenz mit den Resten Y und Z hat den „Gesamt-hydrophob-hydrophil-Charakter" des Nonapeptids zu erhalten, um eine optimale Hormonaktivität zu gewährleisten.

Gleichzeitig wird durch diesen Austausch der Methioninreste durch die Bausteine Y bzw. Z eine Erhöhung der Stabilität der Nonapeptide erzielt (Oxidation der Methionin-zum Methionin-S-oxidresten führt zum Verlust der biologischen Aktivität!) und eine Synthesevereinfachung herbeigeführt.

Die Besetzung der Position 25 (d.i. der Aminosäurerest X) mit einer stark basischen Aminosäure, z.B. in Analogie zur Naturstoffsequenz mit Arginin, aber auch mit den Homo- und Norverbindungen dieser Aminosäure oder anderer stark basischer Aminosäuren, erhöht die Stabilität der Tyrosin-O-sulfatgruppierung in den Nonapeptiden erheblich. Herstellung, Aufarbeitung und Lagerung der hormonaktiven Peptide wird damit erheblich erleichtert.

In der DE-A Nr. 1 800 129 werden biologisch aktive Hexa-, Hepta-, Octa- und Nonapeptide beschrieben, welche in 7-Stellung Tyrosinsulfat enthalten; die 8-Stellung bei dem Nonapeptid wird dabei von Glutaminsäure, die 9-Stellung von Pyroglutaminsäure eingenommen, die sich im Peptidverband neutral verhält.

In der DE-A Nr. 2 037 697 werden Heptapeptide mit biologischer Aktivität beschrieben, bei denen gegenüber den in gleicher Weise aktiven Polypeptiden der DE-A Nr. 1 800 129 wegen der aktivitätsmindernden Instabilität der Tyrosinsulfatgruppierung diese durch eine eine p-Sulphonylphenylgruppe enthaltende Einheit ersetzt wurde.

In der DE-A Nr. 1 922 185 werden Polypeptide mit Cholecystokininaktivität offenbart, welche Dodekapeptide darstellen.

In der DE-A Nr. 1 935 402 werden Octa-, Deka- und Dodekapeptide mit Cholecystokininaktivität offenbart.

In der DE-A Nr. 2 256 445 werden Heptapeptide mit Gastrinwirkung beschrieben, die überhaupt keine Tyrosin-O-sulfatgruppierung aufweisen.

In der DE-A Nr. 2 727 048 wird ein modifiziertes Dekapeptid mit ceruletidanaloger biologischer Aktivität offenbart.

Die Herstellung der erfindungsgemässen Peptide erfolgt nach an sich bekannter Weise, wie beispielsweise beschrieben von L. Moroder, L. Wilschowitz, E. Jaeger, S. Knof, P. Thamm und E. Wünsch in: „Hormonal Receptors in Digestive Tract Physiology" (G. Rosselin et al. eds.), Elsevier/North-Holland Biomedical Press, Amsterdam 1979, S. 129-135. S. ferner L. Moroder, L. Wilschowitz, E. Jaeger, S. Knof, P. Thamm und E. Wünsch (1979), „Hoppe Seyler's Z. Physiol. Chem." 360, 787-790).

Ein besonders vorteilhaftes Verfahren zur Herstellung der erfindungsgemässen Peptide ist dadurch gekennzeichnet, dass man zur Einführung des Aminosäurerestes 27 ein N-Acyltyrosin in einem polaren organischen Lösungsmittel mit überschüssigem Pyridin-$SO_3$ umsetzt, die erhaltene Lösung mit Wasser extrahiert, aus der wässerigen Phase das Bariumsalz des N-Acyltyrosin-O-sulfats durch Zusatz einer löslichen Bariumverbindung fällt, gegebenenfalls die Acylgruppe in üblicher Weise abspaltet und das so erhaltene Tyrosin-O-sulfatbariumsalz oder dessen Acylderivat in an sich bekannter Weise nach üblichen Peptidsynthesemethoden weiterverarbeitet.

Als Zwischenprodukt wird dabei Tyrosin-O-sulfatbariumsalz und/oder ein Acylderivat davon, vorzugsweise N-Carbobenzoxytyrosin-O-sulfatbariumsalz verwendet.

Tyrosin-O-sulfat wurde als Metabolit im Urin von Säugetieren und später auch als Eiweissbaustein in verschiedenen biologisch aktiven Peptiden und Proteinen, wie z.B. den Gastrinen, entdeckt. In den biologisch interessanten Peptidhormonen Cholecystokininpankreocymin und Caerulein stellt dieser Aminosäurerest darüber hinaus eine essentielle Gruppierung für die biologische Aktivität dar. Die Synthese solcher biologisch aktiver tyrosin-O-sulfathaltiger Peptide bzw. Proteine ist daher ein wichtiges Ziel der biochemischen, insbesondere peptidchemischen Forschung und der Arzneimittelentwicklung.

Tyrosin-O-sulfat enthaltende biologisch aktive Peptide wurden bisher überwiegend durch nachträgliche Sulfatisierung des Phenolrests im Peptidverband mittels konzentrierter Schwefelsäure oder Pyridin/$SO_3$-Komplex hergestellt. Diese Methoden haben jedoch den Nachteil, dass in störendem Umfang Nebenreaktionen auftreten, wie Sulfonierung des Phenolkerns, des Indol- oder Imidazolrests einerseits oder Umsetzungen mit der Methioninthioäthergruppierung, der Argininguanido- und der primären Amidofunktion andererseits [„J. Am. Chem. Soc." *68*, 1024 und 1031 (1946)]. Die zusätzlichen, leicht sulfatisierbaren Hydroxyl- und Aminofunktionen mussten geschützt werden.

Dieser Nachteil liesse sich vermeiden, wenn es gelänge, derartige aktive Peptide mit Tyrosin-O-sulfat als Startmaterial aufzubauen. Dadurch würde es möglich, die durch die erwähnten Nebenreaktionen bedingten Beschränkungen bei der Peptidsynthese zu vermeiden. Es wurde zwar bereits über einen Versuch berichtet, mit Tyrosin-O-sulfat in Form des Kaliumsulfats derartige Peptide aufzubauen, jedoch wurden weder experimentelle Angaben hierüber noch die Resultate bekannt [„Experientia" *28*, 7 (1972)].

Mit Hilfe des Tyrosin-O-sulfatbariumsalzes und dessen N-Acylderivate mit in der Peptidchemie üblichen Acylschutzgruppen, vorzugsweise des N-Carbobenzoxytyrosin-O-sulfatbariumsalzes gelingt es, die geschilderten Nachteile zu beseitigen und tyrosin-O-sulfathaltige Verbindungen zur Verfügung zu stellen, welche sich als Startmaterial für die synthetische Herstellung von Peptiden eignen. Aufgrund ihrer Löslichkeitseigenschaften erleichtern sie die Isolierung der damit hergestellten Peptide.

Tyrosin-O-sulfatbariumsalz besitzt die Formel H-Tyr-($SO_3Ba_{1/2}$)-OH, die genannte Carbobenzoxyverbindung die Formel B-Tyr-($SO_3Ba_{1/2}$)-O-$Ba_{1/2}$. In diesen Formeln bedeutet Tyr Tyrosin und B die Carbobenzoxygruppe. Die verwendeten Abkürzungen für die Aminosäurereste entsprechen hier und in der folgenden Beschreibung den Regeln von Houben-Weyl, „Methoden der organischen Chemie", 4. Aufl., Bd. 15/1, S. 20, Verlag Thieme, Stuttgart (1974). Weitere, in der Peptidchemie übliche Acylschutzgruppen sind in Houben-Weyl, *loc. cit.*, aufgeführt. Als Beispiele seien genannt N-t-Butyloxycarbonyl-, N-Fluorenyloxycarbonyl- und N-2-Nitrophenylsulfenyltyrosin-O-sulfatbariumsalz.

Das Tyrosin-O-sulfatbariumsalz wird dadurch hergestellt, dass man ein N-Acyltyrosin in einem polaren organischen Lösungsmittel oder Lösungsmittelgemisch mit überschüssigem Pyridin-$SO_3$ umsetzt, die erhaltene Lösung mit Wasser extrahiert, aus der wässerigen Phase das Bariumsalz des N-Acyltyrosin-O-sulfats durch Zusatz einer löslichen Bariumverbindung fällt und gegebenenfalls die Acylgruppe abspaltet. Bevorzugt wird als N-Acylgruppe die Carbobenzoxygruppe, welche hydrierend abgespalten wird.

Als polares organisches Lösungsmittel wird Pyridin oder ein Pyridin/Dimethylformamid-Gemisch bevorzugt. Es können jedoch auch andere schwach basische oder neutrale polare organische Lösungsmittel bzw. Gemische verwendet werden. Der Überschuss an Pyridin/$SO_3$-Komplex ist unkritisch, zweckmässig wird eine 2- bis 6fache, vorzugsweise eine 3- bis 5fache Menge, bezogen auf $SO_3$-Äquivalent eingesetzt. Die Umsetzung wird zweckmässig bei Temperaturen zwischen etwa 0°C und dem Siedepunkt des Lösungsmittels durchgeführt, bevorzugt werden Temperaturen zwischen etwa Zimmertemperatur und 80°C. Nach Beendigung der Umsetzung wird überschüssiger Pyridin/$SO_3$-Komplex durch Kühlung abgeschieden und aus der Lösung abgetrennt. Durch Einengung des Filtrats lassen sich zusätzliche Mengen an Komplex abtrennen. Anschliessend wird mit Wasser verdünnt, Verunreinigungen werden mit organischen Lösungsmitteln, wie z.B. Essigester, extrahiert, und anschliessend wird der wässerigen Phase eine geeignete lösliche Bariumverbindung, vorzugsweise Bariumhydroxid, zugesetzt. Zweckmässig wird dabei ein 2- bis 3facher Überschuss zugesetzt.

Durch $CO_2$-Zusatz lässt sich überschüssiges Bariumhydroxid abtrennen, wobei ein Sauerwerden der Lösung durch Zugabe eines schwachen Alkalis, vorzugsweise einer organischen Base, wie Pyridin, vermieden wird. Nach Entfernung des Niederschlags wird das acylgruppenhaltige Bariumsalz des Tyrosin-O-sulfats gewonnen. Es kann als solches für die Peptidsynthese verwendet oder in das Tyrosin-O-sulfatbariumsalz überführt werden. Die Abspaltung der Acylgruppe erfolgt in der in der Peptidchemie üblichen Weise, z.B. bei der Carbobenzoxygruppe, durch Hydrierung in Gegenwart von Palladium als Katalysator, zweckmässig in Dimethylformamid als Reaktionsmedium.

Die neuen Bariumsalze erweisen sich für den totalsynthetischen Aufbau tyrosin-O-sulfathaltiger Peptide bzw. Proteine als besonders geeignet, da sie die Isolierbarkeit der damit hergestellten Peptidderivate positiv beeinflussen. Überraschenderweise ergab sich dabei, dass die üblichen Methoden der Peptidsynthese hierbei angewendet werden können, ohne dass die O-Sulfatgruppe abgespalten wird. Dies gilt unter anderem auch für die Abspaltung der Schutzgruppen, beispielsweise von Schutzgruppen auf tert.-Butanolbasis unter sauren Bedingungen. Dies war nicht zu erwarten, da bisher stets auf die Säureinstabilität des Tyrosin-O-sulfats hingewiesen wurde. Typische Beispiele für Peptidsynthesemethoden, die in Gegenwart der erfindungsgemässen Verbindungen durchgeführt werden können, ohne letztere zu zersetzen, sind die Kondensation nach der Dicyclohexylcarbodiimid/1-Hydroxybenzotriazol-Methode (DCCD/HOBT-Methode), die Methoden über gemischte Anhydride mit z.B. Pivaloylchlorid, Chlorameisensäureäthylester oder Isobutylester. Ein anderes Beispiel für eine geeignete Synthesemethode ist die Dicyclohexylcarbodiimid/N-Hydroxysuccinimid-Methode.

Die besondere Brauchbarkeit der erfindungsgemässen Zwischenprodukte wird mit Hilfe des benzyloxycarbonylgruppenhaltigen Bariumsalzes am Beispiel der Synthese des Nonapeptids der Sequenz H-Arg-Asp-Tyr($SO_3H$)-Thr-Gly-Trp-Leu-Asp-Phe-$NH_2$ demonstriert. Dabei wurde die erfindungsgemässe Bariumverbindung nach der DCCD/HOBT-Methode an H-Thr (tBu)-Gly-Trp-Leu-Asp(OtBu)-Phe-$NH_2$ aufkondensiert und führte eindeutig zu B-Tyr($SO_3Ba_{1/2}$)-Thr(tBu)-Gly-Trp-Leu-Asp(OtBu)-Phe-$NH_2$ (III) mit 70% Ausbeute. Die hydrogenolytische Abspaltung des Benzyloxycarbonylrestes von III unter üblichen Hydrierungsbedingungen ergab 96% Ausbeute an H-Tyr($SO_3Ba_{1/2}$)-Thr(tBu)-Gly-Trp-Leu-Asp(OtBu)-Phe-$NH_2$ (IV). Eine anschliessende Aufstockung des aminofreien Tyrosin-O-sulfatpeptidderivats IV mit B-Arg($B_2$)-Asp-(OtBu)-OH nach der DCCD/HOSU-Methode zu B-Arg($B_2$)-Asp(OtBu)-Tyr($SO_3Ba_{1/2}$)-Thr-(tBu)-Gly-Trp-Leu-Asp(OtBu)-Phe-$NH_2$ (V) verlief ebenfalls mit 96% Ausbeute. Die hydrogenolytische Abspaltung der drei Benzyloxycarbonylgruppen vom aminoendständigen Argininrest bei etwa pH 6 lieferte das gewünschte Nonapeptidderivat VI, d.h. H-Arg-Asp(OtBu)-Tyr($So_3H$)-Thr(tBu)-Gly-Trp-Leu-Asp(OtBu)-Phe-$NH_2$ mit 83% Ausbeute.

Trotz der vielfach in der Literatur angegebenen beachtlichen Säureinstabilität von Tyrosin-O-sulfat und seiner Peptide gelang auch die letzte Stufe der Herstellung des CCK-PZ-Nonapeptidanalogons, d.h. die Abspaltung der Schutzgruppen auf tert.-Butanolbasis mit überraschend positivem Ergebnis. Die befürchtete gleichzeitige teilweise Spaltung der Sulfathalbestergruppierung trat nicht auf. Für die Abspaltung der Schutzgruppen eignen sich die hierfür bekannten sauren Mittel, bevorzugt wird etwa 70 bis etwa 90%ige Trifluoressigsäure. Zweckmässig wird dabei ein Kationenfänger, wie z.B. 2-Methylindol, zugesetzt.

Die folgenden Beispiele erläutern die Erfindung weiter.

*Beispiel 1:*

*B-Tyr(SO₃Ba₁/₂)-O-Ba₁/₂·3H₂O (I)*

12,7 g (40,3 mmol) B-Tyr-OH in Pyridin werden mit 25,8 g (161,1 mmol) Pyridin/$SO_3$-Komplex versetzt. Nach Erwärmen der Suspension auf 60° C und Rühren bei dieser Temperatur bis sich der Komplex gelöst hat (ca. ½ h) wird auf 0° C gekühlt und filtriert; das Filtrat wird im Vakuum eingeengt und nochmals filtriert, um den überschüssigen Komplex abzutrennen. Nun wird die Lösung mit Wasser verdünnt und zweimal mit Essigester extrahiert, die abgetrennte wässerige Phase wird mit Stickstoff gesättigt und je nach Menge des oben zurückgewonnenen Komplexes mit 2 bis 3 Äquivalenten Bariumhydroxid versetzt.

Der Niederschlag wird abgesaugt und der Überschuss an Bariumhydroxid als $BaCO_3$ durch Einleiten von $CO_2$ entfernt. Das Absinken des pH unter 7 wird hierbei durch Zugabe von Pyridin verhindert.

Nach Filtration engt man die Lösung auf ca. 100 ml ein und fällt das Produkt mit Äthanol aus. Chromatographisch rein in n-BuOH/AcOH/$H_2O$/Essigester (3:1:1:5).
$[\alpha]_{546}^{20}$: + 22,7° bzw. $[\alpha]_D^{20}$: + 18,9° (c = 1, in DMF).
Ausbeute: 21,34 g (91% der Theorie).
$C_{17}H_{15}NO_8SBa \cdot 3H_2O$ (584,80)
Berechnet: C 34,92  H 3,62  N 2,39  Ba 25,88%
Gefunden: C 34,87  H 3,10  N 2,20  Ba 23,30%

Ber. Rückstand 39,9% (als $BaSO_4$)
Gef. Glührückstand 40,5% (als $BaSO_4$)

*Beispiel 2:*

*H-Tyr(SO₃Ba₁/₂)-OH·H₂O (II)*

5 g (8,55 mmol) B-Tyr($SO_3Ba_{1/2}$)-O-$Ba_{1/2}$·$3H_2O$ werden wie üblich in Gegenwart von Palladium als Katalysator in Dimethylformamid hydriert. Nach beendeter Reaktion wird das Filtrat im Vakuum eingedampft und der Rückstand in Wasser gelöst. Man filtriert vom Unlöslichen ab und fällt das Produkt mit Äthanol aus. Dieses wird im Wasser gelöst und der pH-Wert der Lösung wird durch Einleiten von $CO_2$ auf 6,5 eingestellt. Der Niederschlag ($BaCO_3$) wird abfiltriert, das Filtrat eingeengt und letztlich wird das Produkt mit Äthanol ausgefällt.

Chromatographisch rein in n-BuOH/AcOH/$H_2O$/Essigester (3:1:1:5); Schmelzpunkt 232° C.
$[\alpha]_D^{20}$: −24,8° bzw. $[\alpha]_{546}^{20}$: −29,6° (c = 1, in DMF/$H_2O$; 95:5 V/V).
Ausbeute: 2,6 g (84% der Theorie).

$C_9H_{10}NO_6SBa_{0,5} \cdot H_2O$ (346,94)
Berechnet:  C 31,15  H 3,48  N 4,04%
Gefunden:  C 30,99  H 3,12  N 3,96%

Rückstand:  berechnet: 32,18%; gefunden: 33,64%

*Beispiel 3:*

A. *B-Tyr(SO₃Ba₁/₂)-Thr(tBu)-Gly-Trp-Leu-Asp(OtBu)-Phe-NH₂ (III)*

Zu einer Lösung von 0,28 g (0,48 mmol) B-Tyr-($SO_3Ba_{1/2}$)-O·$3H_2O$ in Dimethylformamid wird bei 0° C 0,62 ml (0,48 mmol) einer 0,77 n HCl-Lösung in Dioxan zugetropft. Unter Rühren gibt man 0,35 g (0,40 mmol) H-Thr(tBu)-Gly-Trp-Leu-Asp(OtBu)-Phe-$NH_2$·$H_2O$, 70 mg (0,52 mmol) 1-Hydroxybenzotriazol und letztlich bei −10° C 99 mg (0,48 mmol) Dicyclohexylcarbodiimid zu. Nach 6 h bei −4° C und 6 h bei Raumtemperatur wird das Lösungsmittel im Vakuum entfernt und der Rückstand mit Äther digeriert. Das Rohprodukt wird in Dimethylformamid aufgenommen; man filtriert vom Unlöslichen und fällt das Produkt mit Wasser aus. Das Produkt wird nochmals aus Methanol nach Abfiltration vom Unlöslichen mit Äther gefällt.

Chromatographisch rein in n-BuOH/AcOH/H$_2$O/Essigester (3:1:1:5); Schmelzpunkt: 168° C (Zers.).

$[\alpha]_{546}^{20}$: −22,7° (c = 1, in Dimethylformamid), $[\alpha]_D^{20}$: −18,8°.

C$_{61}$H$_{78}$N$_9$O$_{16}$SBa$_{0,5}$ (1294,11)

| | | | |
|---|---|---|---|
| Berechnet: | C 56,62 | H 6,08 | N 9,79% |
| Gefunden: | C 56,64 | H 6,21 | N 9,87% |

B.  *H-Tyr(SO$_3$Ba$_{1/2}$)-Thr(tBu)-Gly-Trp-Leu-Asp (OtBu)-Phe-NH$_2$/1H$_2$O· 1 DMF*

4,07 g (3,14 mmol) B-Tyr(SO$_3$)-Thr(tBu)-Gly-Trp-Leu-Asp(OtBu)-Phe-NH$_2$/1/2 Ba werden in Dimethylformamid in Gegenwart von Palladium wie üblich hydriert. Nach Entfernung des Katalysators wird das Lösungsmittel im Vakuum abgezogen und der Rückstand mit Äther verrieben.

Chromatographisch rein in n-BuOH/AcOH/H$_2$O/Essigester (3:1:1:5); Schmelzpunkt 178° C (Zers.).

$[\alpha]_D^{20}$: −29,85° bzw. $[\alpha]_{546}^{20}$: −36° (c = 1, in DMF). Ausbeute: 3,72 g (96% der Theorie).

C$_{53}$H$_{72}$N$_9$O$_{14}$SBa$_{0,5}$·1H$_2$O·1 DMF (1235,10)

| | | | |
|---|---|---|---|
| Berechnet: | C 54,46 | H 6,61 | N 11,34% |
| Gefunden: | C 54,70 | H 6,73 | N 11,34% |

C.  *B-Arg(B$_2$)-Asp(OtBu)-Tyr(SO$_3$Ba$_{1/2}$)-Thr-(tBu)-Gly-Trp-Leu-Asp(OtBu)-Phe-NH$_2$· 2H$_2$O*

3 g (2,43 mmol) H-Tyr(SO$_3$Ba$_{1/2}$)-Thr(tBu)-Gly-Trp-Leu-Asp(OtBu)-Phe-NH$_2$·1H$_2$O und 2,18 g (2,92 mmol) B-Arg(B$_2$)-Asp(OtBu)-OH werden in Dimethylformamid mit 0,36 g (3,16 mmol) N-Hydroxysuccinimid und bei −20°C mit 0,63 g (3,04 mmol) Dicyclohexylcarbodiimid versetzt. Nach 24 h bei 4° C und 24 h bei Raumtemperatur wird die Reaktionsmischung vom ausgefallenen Harnstoff abfiltriert und im Vakuum eingeengt. Der Rückstand wird zweimal aus Dimethylformamidäther umgefällt.

Chromatographisch rein in n-BuOH/AcOH/H$_2$O/Essigester (3:1:1:5); Schmelzpunkt 190 bis 195° C.

$[\alpha]_D^{20}$: −14,3° bzw. $[\alpha]_{546}^{20}$: −17,3° (c = 1, in DMF).

D.  *H-Arg-Asp(OtBu)-Tyr(SO$_3$H)-Thr(tBu)-Gly-Trp-Leu-Asp(OtBu)-Phe-NH$_2$*

3,7 g (1,9 mmol) B-Arg(B$_2$)-Asp(OtBu)-Tyr-(SO$_3$Ba$_{1/2}$)-Thr(tBu)-Gly-Trp-Leu-Asp(OtBu)-Phe-NH$_2$·2H$_2$O wurden wie üblich in Gegenwart von Palladium unter Zusatz von 26,34 ml 0,143 n HCl in MeOH (theor. 26,87 ml) bei pH 6 in Dimethylformamid hydriert.

Nach Entfernung des Katalysators wurde das Produkt mit triäthylaminhaltigem Wasser gefällt. Der Niederschlag wurde aus Dimethylformamid/Wasser umgefällt und sorgfältig mit Wasser gewaschen.

Chromatographisch rein in n-BuOH/AcOH/H$_2$O/Essigester (3:1:1:5); Schmelzpunkt 208° C (Zers.).

$[\alpha]_{546}^{20}$: −25,5° bzw. $[\alpha]_D^{20}$: −21,2° (c = 1, in DMF).

Ausbeute: 2,24 g (83% der Theorie).

C$_{67}$H$_{97}$N$_{14}$O$_{18}$S (1418,7)

| | | | |
|---|---|---|---|
| Berechnet: | C 56,72 | H 6,89 | N 13,82% |
| Gefunden: | C 56,63 | H 6,92 | N 13,47% |

*Beispiel 4:*

Nach dieser Methode konnte ferner folgende Verbindung hergestellt werden:

*H-Arg-Asp-Tyr(SO$_3$H)-Thr-Gly-Trp-Nle-Asp-Phe-NH$_2$:*

Aminosäurenanalyse (berechnete Werte in Klammern) des sauren Hydrolysats (6M HCl/110° C/24 h unter Zusatz von 2,5% Thioglykolsäure):

Arg 1,00(1) Asp 1,92(2) Tyr 1,00(1) Thr 1,03(1) Gly 0,98(1) Trp 0,91(1) Nle 1,02(1) Phe 1,00(1); des AP-M Abbaus: Arg 1,00(1) Asp 1,96(2) Tyr-(SO$_3$H) 0,98(1) Thr 1,05(1) Gly 1,00(1) Trp 1,00(1) Nle 1,01(1) Phe 1,00(1);

dünnschichtchromatographisch (HPTLC-Fertigplatten Kieselgel 60, Merck AG, Darmstadt) einheitlich in n-Butanol/Essigsäure/Pyridin/Wasser (60:6:40:24); einheitlich nach Hochdruckflüssigkeitschromatographie (Säule: μ-Bondapak C 18; Bluens: 29% Acetonitril +71% 0,01M Ammoniumacetat Puffer, pH 4,0; isokratisch) und trägerfreien Elektrophorese (Kammerelektrolyt: 0,1M Ammoniumacetat Puffer, pH 8,3; Elektrodenabstand: 50 cm bei 1500 V); Tyr/Trp = 0,0 (laut UV-Messung).

**Patentansprüche für die Vertragsstaaten: CH, DE, FR, GB, LI, SE**

1. Pankreozymincholezystokinin aktive Peptide mit der carboxylendständigen Sequenz des Pankreozymincholezystokinins 25-33, dergestalt, dass der Aminosäurerest 25 eine stark basische Gruppe trägt, der Aminosäurerest 28 einen hydrophilen Charakter vom Typ Hydroxyaminosäuren besitzt und der Aminosäurerest 31 einen stark hydrophoben Charakter vom Typ der Aminosäuren mit aliphatischen Seitenketten besitzt.

2. Peptid nach Anspruch 1, dadurch gekennzeichnet, dass es ein Nonapeptid ist.

3. Peptid nach einem der beiden Ansprüche 1 oder 2, dadurch gekennzeichnet, dass der Aminosäurerest 28 Threonin, Serin oder Hydroxyprolin ist.

4. Peptid nach einem der vorgehenden Ansprüche, dadurch gekennzeichnet, dass der Aminosäurerest 31 Norleucin, Leucin, Norvalin oder α-Aminobuttersäure ist.

5. Nonapeptide der Sequenz H-X-Asp-Tyr-(SO$_3$H)-Y-Gly-Trp-Z-Asp-Phe-NH$_2$ wobei X Arginin, Homoarginin, Norarginin, N$_\epsilon$, N$_\epsilon$-Dialkyllysin, N$_\delta$ oder N$_\delta$-Dialkylornithin, Y Threonin, Serin oder Hydroxyprolin und Z Norleucin, Norvalin oder α-Aminobuttersäure ist.

6. Nonapeptid der Sequenz H-Arg-Asp-Tyr-(SO₃H)-Thr-Gly-Trp-Nle-Asp-Phe-NH₂.

7. Verfahren zur Herstellung der Peptide gemäss einem der Ansprüche 1 bis 6 durch übliche Methoden der Peptidsynthese, dadurch gekennzeichnet, dass man zur Einführung des Aminosäurerestes 27 ein N-Acyltyrosin in einem polaren organischen Lösungsmittel mit überschüssigem Pyridin-SO₃ umsetzt, die erhaltene Lösung mit Wasser extrahiert, aus der wässerigen Phase das Bariumsalz des N-Acyltyrosin-O-sulfats durch Zusatz einer löslichen Bariumverbindung fällt, gegebenenfalls die Acylgruppe in üblicher Weise abspaltet, und das so erhaltene Tyrosin-O-sulfatbariumsalz oder dessen Acylderivat in an sich bekannter Weise nach üblichen Peptidsynthesemethoden weiterverarbeitet.

8. Verfahren nach Anspruch 7, dadurch gekennzeichnet, dass man das Tyrosin-O-sulfatbariumsalz durch Kondensation nach der Dicyclohexylcarbodiimid/1-Hydroxybenzotriazol-Methode weiterverarbeitet.

9. Verfahren nach Anspruch 7, dadurch gekennzeichnet, dass man das Tyrosin-O-sulfatbariumsalz nach der Methode über gemischte Anhydride weiterverarbeitet.

10. Verfahren nach Anspruch 7, dadurch gekennzeichnet, dass man das Tyrosin-O-sulfatbariumsalz nach der Dicyclohexylcarbodiimid/N-Hydroxysuccinimid-Methode weiterverarbeitet.

11. Verfahren nach Anspruch 7 zur Herstellung des Nonapeptids gemäss Anspruch 5, dadurch gekennzeichnet, dass Tyrosin-O-sulfatbariumsalz nach der Dicyclohexylcarbodiimid/1-Hydroxybenzotriazol-Methode an H-Y(tBu)-Gly-Trp-Z-Asp(OtBu)-Phe-NH₂ aufkondensiert wird, aus dem Kondensationsprodukt der Benzyloxycarbonylrest hydrogenolytisch abgespalten wird, das so erhaltene Tyrosin-O-sulfatpeptidderivat mit B-X(B₂)-Asp(OtBu)-OH nach der Dicyclohexylcarbodiimid/N-Hydroxysuccinimid-Methode zu B-X(B₂)-Asp(OtBu)-Tyr(SO₃Ba₁/₂)-Y(tBu)-Gly-Trp-Z-Asp(OtBu)-Phe-NH₂ aufgestockt wird, aus welchem die drei Benzyloxycarbonylgruppen (B) vom aminoendständigen X-Rest hydrogenolytisch abgespalten werden, worauf aus dem so erhaltenen Nonapeptidderivat die tert.-Butanolschutzgruppen in an sich bekannter Weise mit einem sauren Agens, vorzugsweise Trifluoressigsäure, abgespalten werden.

12. Arzneimittel zur Steuerung der Funktion der Gallenblase und zur Steuerung der Enzymsekretion des Pankreas enthaltend ein Peptid gemäss einem der Ansprüche 1 bis 6 neben üblichen Träger- und/oder Hilfsstoffen.

**Patentansprüche für den Vertragstaat AT**

1. Verfahren zur Herstellung Pankreozymincholezystokinin aktiver Peptide mit der carboxylendständigen Sequenz des Pankreozymincholezystokinins 25-33, dergestalt, dass der Aminosäurerest 25 eine stark basische Gruppe trägt, der Aminosäurerest 28 einen hydrophilen Charakter vom Typ Hydroxyaminosäuren besitzt, und der Aminosäurerest 31 einen stark hydrophoben Charakter vom Typ der Aminosäuren mit aliphatischen Seitenketten besitzt, dadurch gekennzeichnet, dass man zur Einführung des Aminosäurerestes 27 ein N-Acyltyrosin in einem polaren organischen Lösungsmittel mit überschüssigem Pyridin-SO₃ umsetzt, die erhaltene Lösung mit Wasser extrahiert, aus der wässerigen Phase das Bariumsalz des N-Acyltyrosin-O-sulfats durch Zusatz einer löslichen Bariumverbindung fällt, gegebenenfalls die Acylgruppe in üblicher Weise abspaltet und das so erhaltene Tyrosin-O-sulfatbariumsalz oder dessen Acylderivat in an sich bekannter Weise nach üblichen Peptidsynthesemethoden weiterverarbeitet.

2. Verfahren nach Anspruch 1 zur Herstellung von Nonapeptiden der Sequenz H-X-Asp-Tyr-(SO₃H)-Y-Gly-Trp-Z-Asp-Phe-NH₂, wobei X Arginin, Homoarginin, Norarginin, Nₑ, Nₑ-Dialkyllysin, N_δ oder N_δ-Dialkylornithin, Y Threonin, Serin oder Hydroxyprolin und Z Norleucin, Leucin, Norvalin oder α-Aminobuttersäure ist.

3. Verfahren nach Anspruch 1 zur Herstellung eines Nonapeptids der Sequenz H-Arg-Asp-Tyr-(SO₃H)-Thr-Gly-Trp-Nle-Asp-Phe-NH₂.

4. Verfahren nach einem der Ansprüche 1 bis 3, dadurch gekennzeichnet, dass man das Tyrosin-O-sulfatbariumsalz durch Kondensation nach der Dicyclohexylcarbodiimid/1-Hydroxybenzotriazol-Methode weiterverarbeitet.

5. Verfahren nach einem der Ansprüche 1 bis 3, dadurch gekennzeichnet, dass man das Tyrosin-O-sulfatbariumsalz nach der Methode über gemischte Anhydride weiterverarbeitet.

6. Verfahren nach einem der Ansprüche 1 bis 3, dadurch gekennzeichnet, dass man das Tyrosin-O-sulfatbariumsalz nach der Dicyclohexylcarbodiimid/N-Hydroxysuccinimid-Methode weiterverarbeitet.

7. Verfahren nach Anspruch 2, dadurch gekennzeichnet, dass Tyrosin-O-sulfatbariumsalz nach der Dicyclohexylcarbodiimid/1-Hydroxybenzotriazol-Methode an H-Y(tBu)-Gly-Trp-Z-Asp(OtBu)-Phe-NH₂ aufkondensiert wird, aus dem Kondensationsprodukt der Benzyloxycarbonylrest hydrogenolytisch abgespalten wird, das so erhaltene Tyrosin-O-sulfatpeptidderivat mit B-X(B₂)-Asp(OtBu)-OH nach der Dicyclohexylcarbodiimid/N-Hydroxysuccinimid-Methode zu B-X(B₂)-Asp(OtBu)-Tyr(SO₃Ba₁/₂)-Y(tBu)-Gly-Trp-Z-Asp(OtBu)-Phe-NH₂ aufgestockt wird, aus welchem die drei Benzyloxycarbonylgruppen (B) vom aminoendständigen X-rest hydrogenolytisch abgespalten werden, worauf aus dem so erhaltenen Nonapeptidderivat die tert.-Butanolschutzgruppen in an sich bekannter Weise mit einem sauren Agens, vorzugsweise Trifluoressigsäure, abgespalten werden.

**Revendications pour l'Etat contractant: AT**

1. Procédé pour la préparation de peptides à activité pancréocyminocholécystokininique ayant, en fin de chaîne carboxylique, la séquence de la pancréocyminecholécystokinine 25-33, en ce sens que le radical acide aminé 25 porte un groupe fortement basique, que le radical acide aminé 28 présente un caractère hydrophile du type des acides hydroxyaminés et que le radical acide aminé 31 possède un caractère fortement hydrophobe du type des acides aminés à chaînes latérales aliphatiques, caractérisé en ce que, pour l'introduction du radical acide aminé 27, on fait réagir une tyrosine N-acylée dans un solvant organique polaire avec un excès de pyridine-$SO_3$, en ce qu'on épuise à l'eau la solution obtenue, on précipite, à partir de la phase aqueuse, le sel de baryum du sulfate de tyrosine-O-N-acylé par addition d'un composé soluble du baryum, on élimine le cas échéant le groupe acyle suivant le mode habituel et on soumet le sel de baryum de sulfate de tyrosine-O ou son dérivé acylé ainsi obtenu à un traitement ultérieur en soi connu suivant des méthodes usuelles de la synthèse des peptides.

2. Procédé selon la revendication 1 pour la préparation de nonapeptides ayant la séquence H-X-Asp-Tyr($SO_3$H)-Y-Gly-Trp-Z-Asp-Phe-$NH_2$ dans laquelle X représente l'arginine, l'homoarginine, la norarginine, une $N_\varepsilon$, $N_\varepsilon$-dialcoyllysine, une $N_\delta$ ou $N_\delta$-dialcoylornithine, Y représente la thréonine, la sérine ou l'hydroxyproline, et Z représente la norleucine, la leucine, la norvaline ou l'acide α-aminobutyrique.

3. Procédé selon la revendication 1 pour la préparation d'un nonapeptide ayant la séquence H-Arg-Asp-Tyr($SO_3$H)-Thr-Gly-Trp-Nle-Asp-Phe-$NH_2$.

4. Procédé selon l'une quelconque des revendications 1 à 3, caractérisé en ce qu'on soumet le sel de baryum de sulfate de tyrosine-O à un traitement ultérieur par condensation suivant la méthode du carbodiimide de dicyclohexyle/1-Hydroxybenzotriazole.

5. Procédé selon l'une quelconque des revendications 1 à 3, caractérisé en ce qu'on soumet le sel de baryum de sulfate de tyrosine-O à un traitement ultérieur suivant la méthode passant par les anhydrides mixtes.

6. Procédé selon l'une quelconque des revendications 1 à 3, caractérisé en ce qu'on soumet le sel de baryum de sulfate de tyrosine-O à un traitement ultérieur suivant la méthode du carbodiimide de dicyclohexyle/N-hydroxysuccinimide.

7. Procédé selon la revendication 2, caractérisé en ce que le sel de baryum de sulfate de tyrosine-O est condensé sur H-Y(tBu)-Gly-Trp-Z-Asp(OtBu)-Phe-$NH_2$ suivant la méthode du carbodiimide/1-hydroxybenzotriazole, le radical benzyloxycarbonyle est éliminé par hydrogénolyse du produit de condensation, le dérivé peptidique de sulfate de tyrosine-O ainsi obtenu est traité par B-X($B_2$)-Asp(OtBu)-OH suivant la méthode du carbodiimide de dicyclohexyle/N-hydroxysuccinimide pour donner B-X($B_2$)-Asp(OtBu)-Tyr($SO_3$$Ba_{1/2}$)-Y(tBu)-Gly-Trp-Z-Asp(OtBu)-Phe-$NH_2$, à partir duquel les trois groupes benzyloxycarbonyle (B) du radical X en fin de chaîne amino sont éliminés par hydrogénolyse, puis les groupes tertiobutanol de protection sont éliminés du dérivé nonapeptidique ainsi obtenu, de façon connue en soi, avec un agent acide, de préférence l'acide trifluoracétique.

## Revendications pour les Etats contractants : CH, DE, FR, GB, LI, SE

1. Peptides à activité pancréocyminocholécystokininique ayant, en fin de chaîne carboxylique, la séquence de la pancréocyminecholécystokinine 25-33, en ce sens que le radical acide aminé 25 porte un groupe fortement basique, que le radical acide aminé 28 présente un caractère hydrophile du type des acides hydroxyaminés et que le radical acide aminé 31 possède un caractère fortement hydrophobe du type des acides aminés à chaînes latérales aliphatiques.

2. Peptide selon la revendication 1, caractérisé en ce qu'il s'agit d'un nonapeptide.

3. Peptide selon l'une des revendications 1 ou 2, caractérisé en ce que le radical acide aminé 28 est la thréonine, la sérine ou l'hydroxyproline.

4. Peptide selon l'une quelconque des revendications 1 à 3, caractérisé en ce que le radical acide aminé 31 est la norleucine, la leucine, la norvaline ou l'acide α-aminobutyrique.

5. Nonapeptides présentant la séquence H-X-Asp-Tyr($SO_3$H)-Y-Gly-Trp-Z-Asp-Phe-$NH_2$ dans laquelle X représente l'arginine, l'hormoarginine, la norarginine, une $N_\varepsilon$, $N_\varepsilon$-dialcoyllysine, une $N_\delta$ ou $N_\delta$-dialcoylornithine, Y représente la thréonine, la sérine ou l'hydroxyproline, et Z représente la norleucine, la norvaline ou l'acide α-aminobutyrique.

6. Nonapeptide présentant la séquence H-Arg-Asp-Tyr($SO_3$H)-Thr-Gly-Trp-Nle-Asp-Phe-$NH_2$.

7. Procédé pour la préparation des peptides selon l'une quelconque des revendications 1 à 6 par des méthodes usuelles de la synthèse des peptides, caractérisé en ce que, pour l'introduction du radical acide aminé 27, on fait réagir une tyrosine N-acylée dans un solvant organique polaire avec un excès de pyridine-$SO_3$, en ce qu'on épuise à l'eau la solution obtenue, qu'on précipite, à partir de la phase aqueuse, le sel de baryum du sulfate de tyrosine-O-N-acylé par addition d'un composé soluble du baryum, qu'on élimine le cas échéant le groupe acyle suivant le mode habituel et qu'on soumet le sel de baryum de sulfate de tyrosine-O, ou son dérivé acylé ainsi obtenu, à un traitement ultérieur, en soi connu, suivant les méthodes usuelles de la synthèse des peptides.

8. Procédé selon la revendication 7, caractérisé en ce qu'on soumet le sel de baryum de sulfate de tyrosine-O à un traitement ultérieur par condensation suivant la méthode du carbodiimide de dicyclohexyle/1-hydroxybenzotriazole.

9. Procédé selon la revendication 7, caractérisé en ce qu'on soumet le sel de baryum de sulfate de tyrosine-O à un traitement ultérieur suivant la méthode passant par les anhydrides mixtes.

13  0 019 115  14

10. Procédé selon la revendication 7, caractérisé en ce qu'on soumet le sel de baryum de sulfate de tyrosine-O à un traitement ultérieur suivant la méthode du carbodiimide de dicyclohexyle/N-hydroxysuccinimide.

11. Procédé selon la revendication 7 pour la préparation du nonapeptide selon la revendication 5, caractérisé en ce que le sel de baryum de sulfate de tyrosine-O est condensé sur H-Y(tBu)-Gly-Trp-Z-Asp(OtBu)-Phe-NH$_2$ suivant la méthode du carbodiimide de dicyclohexyle/1-hydroxybenzotriazole, le radical benzyloxycarbonyle est éliminé par hydrogénolyse du produit de condensation, le dérivé peptidique de sulfate de tyrosine-O ainsi obtenu est traité par B-X(B$_2$)-Asp(OtBu)-OH suivant la méthode du carbodiimide de dicyclohexyle/N-hydroxysuccinimide pour donner B-X(B$_2$)-Asp(OtBu)-Tyr(SO$_3$Ba$_{1/2}$)-Y(tBu)-Gly-Trp-Z-Asp(OtBu)-Phe-NH$_2$ à partir duquel les trois groupes benzyloxycarbonyliques (B) du radical X en fin de chaîne sont éliminés par hydrogénolyse, puis les groupes tertiobutanol de protection sont éliminés du dérivé nonapeptidique ainsi obtenu, de façon connue en soi, avec un agent acide, de préférence l'acide trifluoracétique.

12. Médicament pour le contrôle de la fonction de la vésicule biliaire et pour le contrôle de la sécrétion enzymatique du pancréas, contenant un peptide selon l'une quelconque des revendications 1 à 6, en plus d'excipients et/ou adjuvants usuels.

**Claims for the contracting state: AT**

1. Method for preparing pancreocymincholecystokinin active peptides with the carboxyl-therminal sequence of the pancreocymincholecystokinin 25-33 such that the amino acid radical 25 carries a strongly basic group, the amino acid radical 28 has a hydrophilic character of the hydroxyamino acid type and the amino acid radical 31 has a strongly hydrophobic character of the amino acid type with aliphatic side chains, characterized in that in order to introduce the amino acid radical 27 a N-acyltyrosine is reacted in a polar organic solvent with excess pyridine-SO$_3$, the solution obtained is extracted with water, the barium salt of N-acyltyrosine-O sulphate is precipitated from the aqueous phase by adding a soluble barium compound, if desired the acyl group is split off in a conventional manner, and the tyrosine-O sulphate barium salt thus obtained, or its acyl derivative, is worked up further according to conventional peptide synthesis methods, in a manner known per se.

2. Method according to claim 1 for preparing nonapeptides of the sequence H-X-Asp-Tyr(SO$_3$H)-Y-Gly-Trp-Z-Asp-Phe-NH$_2$, wherein X is arginine, homoarginine, norarginine, N$_\varepsilon$, N$_\varepsilon$-dialkyllysine, N$_\delta$ or N$_\delta$-dialkylornithine, Y is threonine, serine or hydroxyproline, and Z is norleucine, leucine, norvaline or α-aminobutyric acid.

3. Method according to claim 1 for preparing a nonapeptide of the sequence H-Arg-Asp-Tyr(SO$_3$H)-Thr-Gly-Trp-Nle-Asp-Phe-NH$_2$.

4. Method according to one of claims 1 to 3, characterized in that the tyrosine-O sulphate barium salt is worked up by condensation according to the dicyclohexylcarbodiimide/1-hydroxybenzotriazole method.

5. Method according to one of claims 1 to 3, characterized in that the tyrosine-O sulphate barium salt is worked up by the method of mixed anhydrides.

6. Method according to one of claims 1 to 3, characterized in that the tyrosine-O sulphate barium salt is worked up by the dicyclohexylcarbodiimide/N-hydroxysuccinimide method.

7. Method according to claim 2, characterized in that tyrosine-O sulphate barium salt is condensed by the dicyclohexylcarbodiimide/1-hydroxybenzotriazole method onto H-Y(tBu)-Gly-Trp-Z-Asp(OtBu)-Phe-NH$_2$, the benzyloxycarbonyl radical is split off hydrogenolytically from the condensation product, the tyrosine-O sulphate peptide derivative thus obtained is added together with B-X(B$_2$)-Asp(OtBu)-OH according to the dicyclohexylcarbodiimide / N - hydroxysuccinimide method onto B-X(B$_2$)-Asp(OtBu)-Tyr-(SO$_3$Ba$_{1/2}$)-Y(tBu)-Gly-Trp-Z-Asp(OtBu)-Phe-NH$_2$ from which the three benzyloxycarbonyl groups (B) are split off hydrogenolytically from the amino-terminale X radical, following which the tert.-butanol protective groups are split off in a manner known per se with an acid agent, preferably trifluoroacetic acid, from the nonapeptide derivative thus obtained.

**Claims for the contracting states: CH, DE, FR, GB, LI, SE**

1. Pancreocymincholecystokinin active peptides with the carboxylterminal sequence of the pancreocymincholecystokinin 25-33 such that the amino acid radical 25 carries a strongly basic group, the amino acid radical 28 has a hydrophilic character of the hydroxyamino acid type, and the amino acid radical 31 has a strongly hydrophobic character of the amino acid type with aliphatic side chains.

2. Peptide according to claim 1, characterized in that it is a nonapeptide.

3. Peptide according to claim 1 or 2, characterized in that the amino acid radical 28 is threonine, serine or hydroxyproline.

4. Peptide according to one of the preceding claims, characterized in that the amino acid radical 31 is norleucine, leucine, norvaline or α-aminobutyric acid.

5. Nonapeptides of the sequence H-X-Asp-Tyr(SO$_3$H)-Y-Gly-Trp-Z-Asp-Phe-NH$_2$, wherein X is arginine, homoarginine, norarginine, N$_\varepsilon$, N$_\varepsilon$-dialkyllysine, N$_\delta$ or N$_\delta$-dialkylornithine, Y is threonine, serine or hydroxyproline, and Z is norleucine, norvaline or α-aminobutyric acid.

6. Nonapeptide of the sequence H-Arg-Asp-Tyr(SO$_3$H)-Thr-Gly-Trp-Nle-Asp-Phe-NH$_2$.

7. Method for preparing the peptides according to one of claims 1 to 6 by conventional methods of peptide synthesis, characterized in that in order to introduce the amino acid radical 27 a N-acyltyrosine is reacted in a polar organic solvent with excess pyridine-$SO_3$, the solution obtained is extracted with water, the barium salt of N-Acyltyrosine-O sulphate is precipitated from the aqueous phase by adding a soluble barium compound, if desired the acyl group is split off in a conventional manner, and the tyrosine-O sulphate barium salt thus obtained or its acyl derivative is worked up further according to conventional peptide synthesis methods, in a manner known per se.

8. Method according to claim 7, characterized in that the tyrosine-O sulphate barium salt is worked up by condensation according to the dicyclohexylcarbodiimide/1-hydroxybenzotriazole method.

9. Method according to claim 7, characterized in that the tyrosine-O sulphate barium salt is worked up according to the method of mixed anhydrides.

10. Method according to claim 7, characterized in that the tyrosine-O sulphate barium salt is worked up by the dicyclohexylcarbodiimide/N-hydroxysuccinimide method.

11. Method according to claim 7 for preparing the nonapeptide according to claim 5, characterized in that tyrosine-O sulphate barium salt is condensed by the dicyclohexylcarbodiimide/1-hydroxybenzotriazole method onto H-Y(tBu)-Gly-Trp-Z-Asp(OtBu)-Phe-$NH_2$, the benzyloxy-carbonyl radical is hydrogenolytically split off from the condensation product, the tyrosine-O sulphate peptide derivative thus obtained is added on with B-X($B_2$)-Asp(OtBu)-OH according to the dicyclohexylcarbodiimide/N-hydroxysuccinimide method to B-X($B_2$)-Asp(OtBu)-Tyr-($SO_3Ba_{1/2}$)-Y(tBu)-Gly-Trp-Z-Asp(OtBu)-Phe-$NH_2$, from which the three benzyloxycarbonyl groups (B) are split off hydrogenolytically from the aminoterminal X radical, following which the tert.-butanol protective groups are split off in a manner known per se with an acid agent, preferably trifluoroacetic acid, from the nonapeptide derivative thus obtained.

12. Medicament for controlling the function of the gall bladder and controlling the enzymatic secretion of the pancreas, containing a peptide according to one of claims 1 to 6 in addition to conventional carriers and/or auxiliary agents.